# EUROPEAN PATENT APPLICATION

(11) **EP 3 278 821 A1**
(43) Date of publication of application: **07.02.2018**
(21) Application number: 16773255.1
(22) Date of filing: 01.04.2016
(51) Int. Cl.: A61M 1/18

(54) **HOLLOW FIBER MEMBRANE BLOOD PURIFYING DEVICE**

(30) Priority: 03.04.2015 JP 2015077175
(71) Applicant: Asahi Kasei Medical Co., Ltd., Chiyoda-ku Tokyo 101-8101 (JP)
(72) Inventor: KOYANO, Toshihiro, Tokyo 101-8101 (JP); ASATSUMA, Keiichi, Tokyo 101-8101 (JP); KOIZUMI, Toshinori, Tokyo 101-8101 (JP)
(74) Representative: dompatent von Kreisler Selting Werner - Partnerschaft von Patent- und Rechtsanwälten mbB
(86) International application number: PCT/JP2016/060973
(87) International publication number: WO 2016/159375

(57) **Abstract**

A hollow fiber membrane blood purifying device (1) including a cylindrical container (2), a hollow fiber membrane bundle (3), a potting resin fixing portion (4) embedding and fixing the hollow fiber membrane bundle 3 at each end (2a, 2b) of the cylindrical container (2), headers (5) having nozzles (6), and ports (7) further has the structure in which each header (5) and the cylindrical container (2) are welded in at least two regions over the circumference. The inside face of the header (5) is in contact with a potting resin fixing portion cut face (4S) that is formed by cutting an outer side portion of the potting resin fixing portion (4) in the length direction of the cylindrical container (2) than the end face of each end (2a, 2b) of the cylindrical container (2), and the potting resin fixing portion cut face (4S) is in a compressively deformed state.

## Description

### Technical Field

The present invention relates to a structure of a hollow fiber membrane blood purifying device to purify blood by using hollow fiber membranes packed in a main body container.

### Background Art

Conventionally, various hollow fiber membrane blood purifying devices have been developed for extracorporeal circulation type blood purification therapies such as hemodialysis, hemodiafiltration, hemofiltration, plasma separation, and plasma component fractionation, and are applied to many blood purification therapies and similar purposes using membrane separation techniques.

A typical hollow fiber membrane blood purifying device is composed of a module prepared as follows: a hollow fiber membrane bundle is packed in a cylindrical main body container having a side with ports; then the hollow fiber membrane bundle is bonded and fixed to the main body container with a potting material such as urethane; and headers are attached to both ends of the main body container. To perform hemodialysis using the hollow fiber membrane blood purifying device, a dialysate is allowed to flow in through an inlet port on the side face of the cylindrical container and to flow out through an outlet port. Concurrently, blood is allowed to flow through a blood inlet header into the hollow fiber membranes and to pass toward a blood outlet header, thereby undergoing the hemodialysis.

Such a hollow fiber membrane blood purifying device is required to be sealed fluid-tightly so as not to leak a liquid from joining portions between the headers and the main body container, and as the joining method between the headers and the main body container, ultrasonic welding is used, for example. The ultrasonic welding is a technique in which ultrasonic vibrations are applied while a portion to be welded of a header is in contact with a portion to be welded of a main body container and thus heat is generated to melt the contact faces of both the members and to join together, and is used as a preferred technique for achieving fluid-tightness/air-tightness (for example, see Patent Document 1).

The hollow fiber membrane blood purifying device is required to be sealed fluid-tightly also between the header and a potting resin fixing portion. Such a structure is achieved as follows, for example: a header is attached to a predetermined position of a main body container, and thus a part of the header (for example, an annularly formed convex portion) comes into pressure contact with a potting resin fixing portion that embeds and fixes a hollow fiber membrane bundle.

### Citation List

### Patent Document

Patent Document 1: WO 2013/146663

### Summary

### Technical Problem

However, even with the structure designed so as to seal between a part of a header and a potting material portion, the sealing between the header and the potting resin fixing portion by contact may be insufficient in some cylindrical hollow fiber membrane blood purifying devices. In such a case, blood can get into a space between a part of the header and the potting resin fixing portion, and the blood can be locally left in a part of the hollow fiber membrane blood purifying device after medical practice. Hence, there is a demand for further improvement.

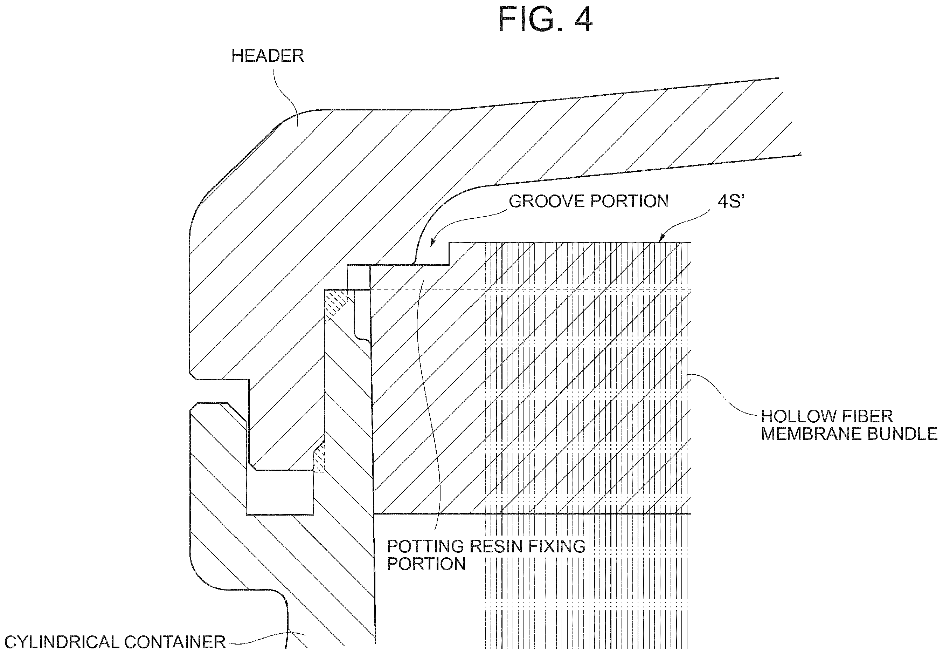

A technique is disclosed to address the above problem. In the technique, only a part of a potting resin fixing portion is cut to form a potting resin fixing portion cut face and an uncut face, and then the potting resin fixing portion uncut face is brought into contact with a header inside face to achieve fluid-tightness. According to the technique, by bringing the potting resin fixing portion uncut face, which is not affected by dimensional variations due to cutting of a potting resin, into contact with the header inside face, fluid-tightness is easily achieved. Meanwhile, a groove portion surrounded by a part of the header and a difference in height between the potting resin fixing portion cut face and the uncut face is formed over the circumference (see Fig. 4). This groove portion becomes a blood retaining portion, which can cause a new problem of activating blood to form blood aggregates.

To overcome the above disadvantages, the present invention aims to provide a hollow fiber membrane blood purifying device that can solve the problems of the formation of blood aggregates due to blood retaining during medical practice and of residual blood after medical practice, by eliminating a level difference of a potting resin fixing portion that can cause blood retaining and activation and by concurrently improving the sealing power between a header and a potting material.

### Solution to Problem

As a result of intensive studies to solve the problems, the inventors of the present invention have found the conditions for optimizing the structure of the hollow fiber membrane blood purifying device and have reached the present invention. In other words, a hollow fiber membrane blood purifying device pertaining to the present invention includes
a cylindrical container having one open end and another open end,
a hollow fiber membrane bundle packed in the cylindrical container,
a potting resin fixing portion embedding and fixing the hollow fiber membrane bundle at each end of the cylindrical container,
a header provided at each end of the cylindrical container and having a nozzle serving as an outlet or inlet of a fluid, and
a port provided on a side of the cylindrical container and serving as an outlet or inlet of a fluid passing through the hollow fiber membrane.

In the hollow fiber membrane blood purifying device,
a shear joint is used as a joint design for welding,
the device has a structure in which the header and the cylindrical container are welded in at least two or more regions,
an inside face of the header is in contact with a potting resin fixing portion cut face that is formed by cutting an outer side portion of the potting resin fixing portion in a length direction of the cylindrical container than an end face of each end of the cylindrical container, and the potting resin fixing portion cut face is in a compressively deformed state.

The hollow fiber membrane blood purifying device has a structure in which a header is in contact with a uniformly cut face of a potting resin fixing portion. This structure eliminates a level difference of the potting resin fixing portion and therefore solves one of the conventional problems of the formation of blood aggregates due to blood retaining during medical practice and residual blood after medical practice.

This structure can simultaneously solve the other problem of residual blood in a hollow fiber membrane blood purifying device after medical practice due to insufficient fluid-tightness. In other words, according to the hollow fiber membrane blood purifying device of the present invention, a shear joint is used as the joint design for welding and enables control of a welding depth. Hence, by bringing the inside face of a header into uniform contact with a potting resin fixing portion cut face over the circumference independent of the height of a potting resin fixing portion cut face, the potting resin fixing portion forms a compressively deformed state. On this account, the inside face of the header is sufficiently in close contact with the potting resin fixing portion over the circumference, thereby giving a hollow fiber membrane blood purifying device in a fluid-tightly sealed state.

The hollow fiber membrane blood purifying device has a structure in which the header and the cylindrical container are welded in at least two or more regions, but the welding structure may be continuous welding over the circumference in the circumferential direction of the cylindrical container or may be partial or intermittent (discontinuous) welding along the circumferential direction of the cylindrical container. Here, the device preferably has a structure of welding over the circumferential direction in at least one or more regions. Moreover, a hollow fiber membrane blood purifying device including a welding portion that is welded over the circumferential direction of the cylindrical container has excellent pressure resistivity and thus is preferred. Meanwhile, when the structure of partial or intermittent (discontinuous) welding in the circumferential direction of the cylindrical container is employed, the application of excess welding energy is not needed, and such a structure is preferred from the viewpoint of production efficiency. As long as sealing properties and pressure resistivity are sufficiently ensured, the welding structure of continuous welding over the circumferential direction of a cylindrical container may be used in combination with the welding structure of partial or intermittent (discontinuous) welding along the circumferential direction of the cylindrical container.

In the hollow fiber membrane blood purifying device, the welding portion close to the region where blood flows is preferably welded over the circumferential direction.

It is preferable that the welded area include a continuous or discontinuous area over a circumference on a side face on an outer periphery of the cylindrical container and on the inside face of the header and a continuous or discontinuous area over a circumference on the end or a side face of each end of the cylindrical container and on the inside face of the header.

It is preferable that a contact face on the inside face of the header in contact with the potting resin fixing portion cut face have a flat face extending outwardly from an inner peripheral edge along a face substantially parallel with the potting resin fixing portion cut face. In this structure, the contact face is in planar contact with the cut face, and thus the structure easily achieves fluid-tightness due to the contact between the potting resin fixing portion cut face and the header inside face as compared with dot-like or linear contact, and is preferred.

It is also preferable that, in a longitudinal section where the header and the cylindrical container are welded, a total sum of a cross-sectional area where the header inside face and the cylindrical container are welded be 0.05 to 1.25 mm².

In the hollow fiber membrane blood purifying device, it is also preferable that, in a longitudinal section where the header and the cylindrical container are welded, a contact width between the potting resin fixing portion cut face and the header inside face be 0.3 to 1.5 mm.

It is also preferable that, in a longitudinal section where the header and the cylindrical container are welded, a height from the end face of the cylindrical container to the cut face of the potting resin fixing portion range from 0.1 to 1.5 mm.

In the hollow fiber membrane blood purifying device, it is preferable that a raw material of the cylindrical container and the headers be a polypropylene resin.

In the hollow fiber membrane blood purifying device, it is preferable that the potting resin have a hardness determined by a Shor
e type D durometer of 30 to 70.

In a longitudinal section of the header and the cylindrical container, it is preferable that one of the header and the cylindrical container have a convex portion with a convex shape, the other have a concave portion with a concave shape to which the convex portion with the convex shape is fit, the convex portion have a leading end face width larger than a bottom face width of the concave portion, and at least one side face of inside faces of the concave portion have a shear joint design with a slope that makes the concave portion bottom face width smaller than a concave portion top face width.

### Advantageous Effects of Invention

The present invention eliminates a level difference of a potting resin fixing portion that can cause blood retaining and activation and concurrently improves the sealing power between a header and a potting material, and this structure can solve the problems of the formation of blood aggregates due to blood retaining during medical practice and of residual blood after medical practice.

### Brief Description of Drawings

Fig. 1 is a half cross-sectional view schematically showing the structure of a hollow fiber membrane blood purifying device of the present invention.

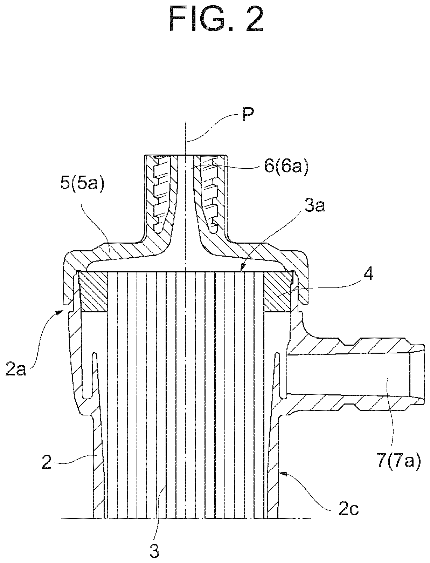

Fig. 2 is an enlarged cross-sectional view showing a part of the hollow fiber membrane blood purifying device.
Fig. 3 is an enlarged view showing a configuration example of a shear joint portion and other portions of the hollow fiber membrane blood purifying device.
Fig. 4 is an enlarged view showing a configuration example of a hollow fiber membrane blood purifying device in Comparative Example 1.
Fig. 5 is a schematic view showing an intermittent (discontinuous) circumferential welding portion between a cylindrical container and a header.

### Description of Embodiments

The structure of the present invention will now be described in detail based on an embodiment shown in drawings.

### <Hollow fiber membrane blood purifying device>

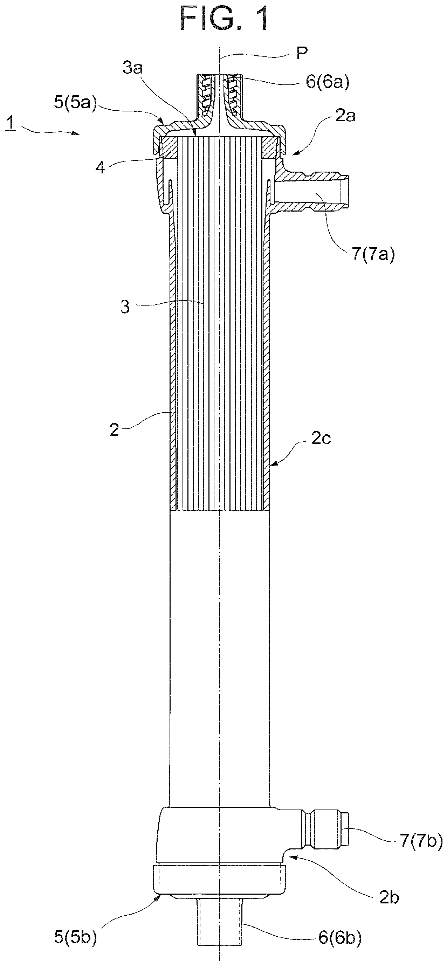

Fig. 1 is a schematic cross-sectional view showing an embodiment of a hollow fiber membrane blood purifying device 1. The hollow fiber membrane blood purifying device 1 includes a cylindrical container 2, a hollow fiber membrane bundle 3, potting resin fixing portions 4, headers 5, nozzles 6, ports 7, welding portions, interference portions 21, 22, and other components.

The cylindrical container (main body container) 2 is a container (cylindrical portion) that is open at both ends, or at one end 2a and the other end 2b, and constitutes the main body of the hollow fiber membrane blood purifying device 1. In Fig. 1, the central axis of the cylindrical container 2 is indicated by sign P. The direction along the central axis P is a length direction of the cylindrical container. The direction around the central axis P as the center is a circumferential direction (circumference direction).

The port 7 is an inlet or outlet of a fluid, and in the hollow fiber membrane blood purifying device 1, a pair of ports 7a, 7b are provided on the side 2c of the cylindrical container 2 (see Fig. 1).

The hollow fiber membrane bundle 3 is packed in the cylindrical container 2 along the length direction and separates impurities in a fluid to be purified. Hollow fiber membranes are made from, as a raw material, polyamide, polypropylene, polymethyl methacrylate, an ethylene-vinyl alcohol copolymer, regenerated cellulose, cellulose acetate, polyacrylonitrile, polyethylene, polysulfone, or polyethersulfone, for example.

When the hollow fiber membrane bundle 3 includes a hydrophobic polymer, a hydrophilic polymer is typically mixed or applied in order to impart hydrophilicity to the membranes.

The hydrophilic polymer means a polymer having affinity with water, especially having blood compatibility. The hydrophilic polymer is exemplified by vinylpyrrolidone-containing polymers or copolymers and alkylene oxide-containing polymers or copolymers. As for the hydrophilic polymer, the vinylpyrrolidone-containing polymer or copolymer means a polymer or copolymer prepared by using vinylpyrrolidone as a monomer.

Other preferred examples of the hydrophilic polymer include polymers and copolymers such as polyvinyl alcohol, polyhydroxyethyl acrylate, polyhydroxypropyl acrylate, polyhydroxybutyl acrylate, polyhydroxyethyl methacrylate, polyhydroxypropyl methacrylate, and polyhydroxybutyl methacrylate.

As the hydrophilic polymer, a single polymer may be used, or a mixture of two or more polymers may be used.

It is also useful to further apply a vitamin to the surface of the hollow fiber membrane bundle. Examples of the vitamin include, but are not limited to, vitamin A, vitamin D, vitamin E, vitamin K, and ubiquinone, and vitamin E is particularly preferred.

The vitamin E is exemplified by α-tocopherol, α-tocopherol acetate, α-tocopherol nicotinate, β-tocopherol, γ-tocopherol, and δ-tocopherol.

In particular, α-tocopherol has various physiological effects including in-vivo antioxidative effect, biomembrane stabilizing effect, and platelet aggregation inhibiting effect, and thus is preferably used.

The potting resin fixing portions 4 are portions that embed the respective ends 3a, 3b of the hollow fiber membrane bundle 3 at the inside of the corresponding ends 2a, 2b of the cylindrical container 2 in the length direction and fix the hollow fiber membrane bundle 3 at the respective ends 2a, 2b of the cylindrical container 2. In the present embodiment, examples of the potting resin include, but are not necessarily limited to, a polyurethane resin, an epoxy resin, and a silicon resin.

The headers 5 (5a, 5b) are provided on the respective ends 2a, 2b of the cylindrical container 2 and face the corresponding ends 3a, 3b of the hollow fiber membrane bundle 3. The headers 5 (5a, 5b) have nozzles 6 (6a, 6b) serving as an inlet or outlet of a fluid.

The cylindrical container 2 and the header 5 are made from any raw material, and the raw material is selected from various thermoplastic resins. For example, as a crystalline resin, examples of the resin include polyethylene resins such as copolymers of ethylene and an α-olefin, low-density polyethylenes, and high-density polyethylenes, and polypropylene resins such as propylene homopolymers, copolymers of propylene and ethylene, and copolymers of propylene, ethylene, and another α-olefin. As an amorphous resin, examples of the resin include polyesters, polycarbonates, polystyrenes, styrene-butadiene copolymers (SBS), and acrylonitrile-butadiene-styrene copolymers (ABS), and these resins may be used singly or as a mixture.

Of them, the preferred resin in the embodiment is a polypropylene resin. Specifically, a random copolymer of propylene and ethylene is preferred in terms of rigidity and heat resistance, and a random copolymer of propylene and ethylene adjusted to have an ethylene content of 1 to 8% by mass is more preferred.

### <Method for producing hollow fiber membrane blood purifying device>

The inside faces of both ends (2a, 2b) of a cylindrical container 2 is subjected to corona or plasma treatment to be hydrophilized; then a hollow fiber membrane bundle 3 is inserted into the cylindrical container 2; to both ends 3a, 3b of the hollow fiber membrane bundle 3, a potting resin is injected; the whole is centrifugally rotated around the center in the length direction of the cylindrical container 2 as the axis before curing; and consequently, a potting resin fixing portions 4 are formed at the respective ends 3a, 3b of the hollow fiber membrane bundle 3 to embed both ends 3a, 3b of the hollow fiber membrane bundle and concurrently to bond and fix the hollow fiber membrane bundle to the cylindrical container 2. For curing, heating may be performed as needed.

After the potting resin is cured to embed and fix the hollow fiber membrane bundle 3 and the cylindrical container 2, an excess potting resin fixing portion 4 is cut and removed to open the end faces (both ends 3a, 3b) of the hollow fiber membrane bundle 3. Then, headers 5 (5a, 5b) are attached to the respective ends (2a, 2b) of the cylindrical container 2 to produce a hollow fiber membrane medical device.

### <Potting resin fixing portion cut face>

After the potting resin is cured to fix the hollow fiber membrane bundle 3 and the cylindrical container 2, an excess potting resin fixing portion 4 is cut and removed, but it has been technically difficult to cut a potting resin fixing portion 4 into a smooth face. On this account, as described in International Publication WO 2013/146663, a smooth potting resin uncut face without cutting is partly formed, and a header is brought into contact with the smooth uncut face to ensure blood sealing properties.

The above method, however, requires the formation of a potting resin fixing portion cut face 4S' concurrently with the formation of a potting resin uncut face, and thus generates a difference in height between the potting resin uncut face and the potting resin fixing portion cut face 4S' (see Fig. 4). After welding of a cylindrical container and a header, the contact of the header inside face with the potting resin fixing portion uncut face forms a groove, which may cause blood retaining and activation. This can cause a problem of forming blood aggregates.

In the present embodiment, an outer side portion of the potting resin fixing portion 4 in the length direction of the cylindrical container 2 than the end face of each end 2a, 2b of the cylindrical container 2 is uniformly cut to form a potting resin fixing portion cut face 4S.

The potting resin fixing portion cut face 4S is preferably smooth not to impair blood sealing properties even when brought into contact with the header 5. The smoothness of the potting resin fixing portion cut face 4S is determined and evaluated as arithmetic average roughness (Ra) in accordance with JIS B0601, and the Ra is preferably 5.0 µm or less. The Ra is more preferably 4.0 µm or less and even more preferably 3.0 µm or less from the viewpoint of blood sealing properties.

In the present embodiment, in order to uniformly cut the potting resin fixing portion 4, a potting resin uncut face where cutting is not partly performed is not formed. On this account, no level difference is formed on the potting resin fixing portion cut face 4S, and thus a hollow fiber membrane blood purifying device including such a structure can suppress blood retaining and activation.

In the embodiment, the height of the potting resin fixing portion cut face 4S is a height from each end (2a, 2b) of the cylindrical container 2 to the corresponding potting resin fixing portion cut face 4S, and is preferably 0.1 mm or more, more preferably 0.2 mm or more, and even more preferably 0.3 mm or more. The height is preferably 1.5 mm or less, more preferably 1.4 mm or less, even more preferably 1.3 mm or less, and particularly preferably 1.2 mm or less.

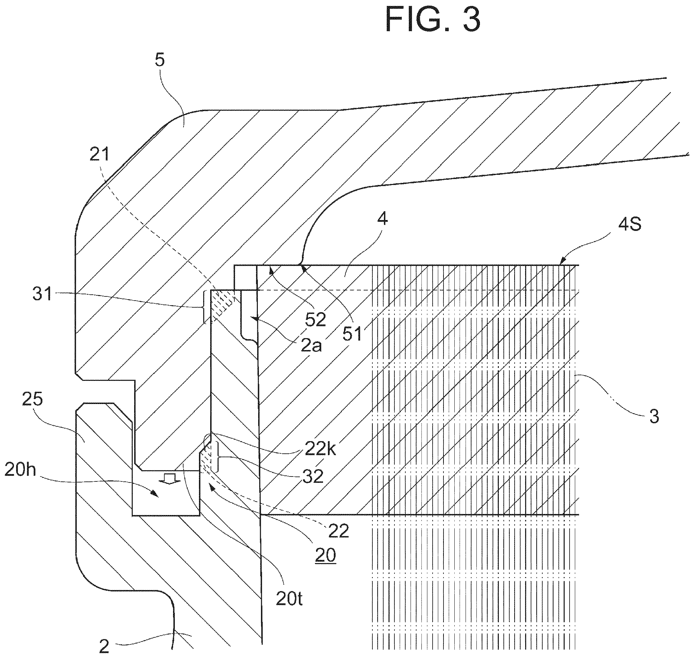

A contact face 52 of the inside face of the header 5 in contact with the potting resin fixing portion cut face 4S has a flat face extending outwardly from an inner peripheral edge 51 along a face substantially parallel with the potting resin fixing portion cut face 4S (see Fig. 3). With this structure, the contact face 52 is in planar contact with the potting resin fixing portion cut face 4S, and thus the structure easily achieves fluid-tightness due to the contact between the potting resin fixing portion cut face 4S and the header inside face, and is preferred.

### <Welding between cylindrical container and header>

In the present embodiment, the cylindrical container 2 and the header 5 are welded in at least two or more regions (i.e. welding portions 31, 32).

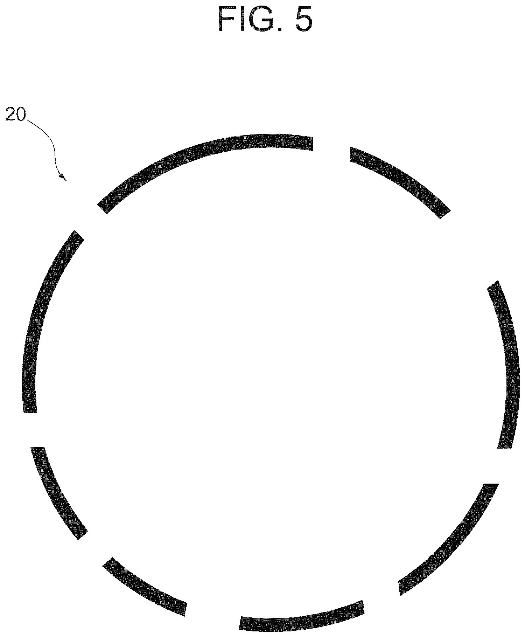

The welding area in the hollow fiber membrane blood purifying device 1 of the embodiment includes two or more areas including a contact area between the outer peripheral side face of the cylindrical container 2 and the inside face of the header 5 and a contact area between the end 2a (2b) or the side face of the end of the cylindrical container 2 and the inside face of the header 5. The contact areas may be in a state of continuous welding over the circumference in the circumferential direction of the cylindrical container 2 or may be in a state of intermittent (discontinuous) welding. Alternatively, continuous welding over the circumferential direction and intermittent (discontinuous) welding may be combined. In an intermittently (discontinuously) welded area, the intermittent interval is not necessarily constant (see Fig. 5).

The state of continuous welding between the cylindrical container 2 and the header 5 over the circumference in the circumferential direction achieves sufficient fluid-tightness/air-tightness in the contact area between the cylindrical container 2 and the header 5, and thus is preferred.

The hollow fiber membrane blood purifying device 1 has the structure in which the header 5 and the cylindrical container 2 are welded in at least two or more regions. The welding structure may be continuous welding over the circumferential direction of the cylindrical container 2 or may be partial or intermittent (discontinuous) welding along the circumferential direction of the cylindrical container 2. Here, the device preferably has a structure of continuous welding over the circumferential direction in at least one or more regions. This welding structure can completely seal the hollow fiber membrane blood purifying device 1 against leakage of flowing blood. A hollow fiber membrane blood purifying device 1 including welding portions 31, 32 that are continuously welded over the circumferential direction of the cylindrical container 2 has excellent pressure resistivity and thus is preferred. Meanwhile, to weld the welding portions 31, 32 over the circumference, high energy is required. To address this, when a structure of partial or intermittent (discontinuous) welding in the circumferential direction of the cylindrical container 2 is employed, the header 5 and the cylindrical container 2 can be welded without the need for excess welding energy. Hence, when the welding structure of continuous welding over the circumferential direction of the cylindrical container 2 is used in combination with the welding structure of partial or intermittent (discontinuous) welding along the circumferential direction of the cylindrical container 2 as long as sealing properties and pressure resistivity are sufficiently achieved, advantages can be obtained.

In the hollow fiber membrane blood purifying device 1, the welding portion 31 close to the region where blood flows is preferably welded over the circumferential direction. This structure can prevent the hollow fiber membrane blood purifying device 1 from leaking blood to outside the system even when blood flows over a sealing portion where the header inside face and the potting resin fixing portion cut face 4S are pressure-bonded.

In addition, the welding area is preferably a contact area between the side face on the outer periphery of the cylindrical container 2 and the inside face of the header 5 and a contact area between the end or the side face of the end of the cylindrical container 2 and the inside face of the header 5. Both of the welding portions 31, 32 may be welding over the circumference.

### <Joint design>

In the present embodiment, a shear joint 20 is used as the joint design for welding in at least two or more contact areas between the cylindrical container 2 and the header 5 (see Fig. 3).

With the shear joint 20, contact faces come close in the same direction as the vibration direction in response to longitudinal vibrations of ultrasonic waves applied along the length direction of the cylindrical container 2 when the cylindrical container 2 and the header 5 are subjected to ultrasonic welding, and thus bubbles are unlikely to be generated on a welding face. Hence, the shear joint has an advantage of excellent fluid-tightness/air-tightness.

Another joint design is exemplified by a butt joint. The butt joint includes a triangular rib called energy director. Ultrasonic vibrations intensively cause a triangular rib portion to expand and contract, thereby generating heat to a resin melting temperature for an extremely short period to enable welding advantageously.

However, when a welding portion has a large volume or a crystalline resin is used, a large energy is required for welding, and thus the butt joint may give uneven welding to fail to achieve sufficient fluid-tightness/air-tightness. Hence, a shear joint is preferably used as the joint design in the present embodiment.

### <Contact between header and potting resin fixing portion cut face during welding>

In the present embodiment, concurrently with the welding between the cylindrical container 2 and the header 5, the potting resin fixing portion cut face 4S comes into contact with the length direction inside face of the header 5 (see Fig. 3). When the length direction inside face of the header 5 comes into contact with the potting resin fixing portion cut face 4S in this manner, the potting resin fixing portion cut face 4S becomes in a compressively deformed state.

The state in which a potting resin fixing portion cut face 4S is compressively deformed is ascertained by the presence of a contact mark of the length direction inside face of the header 5 on the surface of the potting resin fixing portion cut face 4S when the hollow fiber membrane blood purifying device 1 is disassembled into the cylindrical container 2 and the header 5 and the potting resin fixing portion cut face 4S is observed.

As another ascertainment method, the state is ascertained by subjecting a hollow fiber membrane blood purifying device 1 to X-ray-CT measurement to determine the height from a container end to a potting resin fixing portion cut face 4S. Specifically, the state is ascertained when the height from a container end to a potting resin fixing portion cut face 4S in an area where the potting resin fixing portion cut face 4S is not in contact with the contact face 52 of a header 5 is larger than the height from a container end to a potting resin fixing portion cut face 4S in an area where the potting resin fixing portion cut face 4S is in contact with the contact face 52 of the header 5.

In the present embodiment, a shear joint is used as the joint design for welding, and thus when a cylindrical container 2 and a header 5 are welded, welding is performed while the header 5 is pressed to a position where the length direction inside face of the header comes into contact with the potting resin fixing portion cut face 4S.

On this account, the potting resin fixing portion cut face 4S is compressed by the contact with the length direction inside face of the header 5 and is deformed (see Fig. 3), and this can result in sufficiently close contact between the header 5 and the potting resin fixing portion cut face 4S in the hollow fiber membrane blood purifying device 1 to achieve markedly higher sealing power.

### <Contact width between potting resin fixing portion cut face and header inside face>

In the present embodiment, the close contact width between the header 5 and the potting resin fixing portion cut face 4S is preferably 0.3 to 1.5 mm and more preferably 0.4 to 1.5 mm over the circumference.

When the close contact width between the header 5 and the potting resin fixing portion cut face 4S is 0.3 mm or more, the header 5 and the potting resin fixing portion cut face 4S can be uniformly in contact with each other over the circumference, and thus such a condition is preferred. When the width is 1.5 mm or less, the header 5 and the potting resin fixing portion cut face 4S can be in close contact with each other without excess stress, and thus such a condition is preferred.

### <Hardness of potting resin>

In the present embodiment, the potting resin preferably has a hardness determined by a Shore type D durometer of 30 to 70, more preferably 35 to 65, and even more preferably 40 to 60.

When a potting resin having a hardness within the above range is used, and the header 5 is brought into contact with the potting resin fixing portion cut face 4S, the potting resin fixing portion cut face can easily become in a compressively deformed state, which can result in sufficiently close contact between the header 5 and the potting resin fixing portion cut face 4S to achieve markedly higher sealing power.

### <Interference portion>

In the present embodiment, interference portions 21, 22 are formed on the inside face of the header 5 and the end side face of the cylindrical container 2 (see Fig. 3).

The interference portion relates to the joint design, and the interference portions 21, 22 are portions interfering with each other in a process in which a header 5 is placed on an end (2a, 2b) of a cylindrical container 2 and ultrasonic welding is performed.

In the present embodiment, an interference portion 21 is preferably formed at a predetermined area, and in a different area, another interference portion 22 is preferably formed. In other words, interference portions are preferably placed in at least two different areas (see Fig. 3).

The specific shapes and positions of the interference portions 21, 22 are not limited. For example, in the hollow fiber membrane blood purifying device 1 of the embodiment, a part of the inside face of the header 5 is formed into an inward protrusion shape so as to interfere with an end of the cylindrical container 2, thereby forming an interference portion 21.

In the present embodiment, a part of the outside face of the cylindrical container 2 is also formed into an outward protrusion so as to interfere with the side face of the header 5 facing the cylindrical container 2, thereby forming an interference portion 22 (see an area indicated by a broken line in Fig. 3).

An idea of whether the interference portions 21, 22 are formed on the cylindrical container 2 or on the header 5 is merely expedient, and the idea that the interference portions 21, 22 are undoubtedly formed both on the inside face of the header 5 and on the outside face of the cylindrical container 2 in order to interfere with each other can be accepted. In the present specification, it is assumed that the interference portion 21 is formed on the header 5, and the interference portion 22 is formed on the cylindrical container 2 for explanation as described above for convenience.

The interference portions 21, 22 may also have any shape and size, but in a preferred example, the interference portion 21 on the inside face of the header 5 is preferably so formed as to have a trapezoidal cross section having a cross-sectional area of 0.375 to 3.500 mm² in a longitudinal section of the header 5 and the cylindrical container 2, more preferably a trapezoidal cross section having an area of 0.400 to 3.250 mm², and even more preferably 0.450 to 3.000 mm².

The interference portion 22 on the end side face of the cylindrical container 2 is preferably so formed as to have a trapezoidal cross section having a cross-sectional area of 0.180 to 2.375 mm², more preferably a trapezoidal cross section having an area of 0.200 to 2.000 mm², and even more preferably 0.250 to 1.800 mm².

### <Welded cross-sectional area>

In the present embodiment, the cross-sectional area where the header inside face and the cylindrical container are welded is preferably 0.05 to 1.25 mm² in a longitudinal section where the header 5 and the cylindrical container 2 are welded.

The welded cross-sectional area is the area of a region in which a contact face between a header 5 and a cylindrical container 2 is heated and melted by ultrasonic vibrations and fused or deformed in a hollow fiber membrane blood purifying device 1, and can be determined by observing a cross-section in the longitudinal direction of a hollow fiber membrane blood purifying device 1 (see Fig. 3, for example). In the present specification, the welded cross-sectional area means an area of only one cross-section of the right and left cross-sections relative to the central axis P, but does not mean the total area of a pair of right and left cross-sections.

In the present embodiment, the header 5 and the cylindrical container 2 are welded in at least two or more regions including a contact area between the outer peripheral side face of the cylindrical container 2 and the inside face of the header 5 (corresponding to the welding portion 32 in the embodiment) and a contact area between an end of the cylindrical container 2 and the inside face of the header 5 (corresponding to the welding portion 31 in the embodiment), and the welded cross-sectional area is the total sum of the cross-sectional areas of the above two or more regions. As for the method of calculating the total sum of welded cross-sectional areas, in a longitudinal section of a hollow fiber membrane blood purifying device 1, each welded cross-sectional area of at least two or more regions including the contact area between the outer peripheral side face of the cylindrical container 2 and the inside face of the header 5 and the contact area between an end of the cylindrical container 2 and the inside face of the header 5 is calculated, and the total sum of the results are calculated.

In the present embodiment, at least one of the shear joints 20 is preferably positioned in the following configuration (for convenience, the lower side in Fig. 3 is expressed as bottom or bottom face side). In other words, in a longitudinal section of the header 5 and the cylindrical container 2, one side (for example, the header 5 side) preferably has a convex shape of a convex portion 20t, while the other side (for example, the cylindrical container 2 side) preferably has a concave shape of a concave portion 20h to which the convex portion 20t can be fit. In addition, in the longitudinal section, the convex portion 20t preferably has a larger leading end face width (thickness in the diameter direction) than the bottom face width (width in the diameter direction) of the concave portion 20h, and at least one side face of the inside faces of the concave portion 20h preferably has a shear joint 20 with a slope 22k that makes the bottom face width of the concave portion 20h smaller than the top face width of the concave portion 20h (see Fig. 3).

On the outside face of the cylindrical container 2, a flange portion 25 that protrudes outwardly and then bends toward the header 5 in a cross-sectional shape is formed, and this structure allows the convex portion 20t of the header 5 to fit along the length direction of the concave portion 20h.

When at least one shear joint 20 is placed as described above, and ultrasonic welding is started while the convex portion leading end face of the header 5 is in contact with the slope 22k formed on the concave portion of the cylindrical container 2, the header 5 and the cylindrical container 2 can be efficiently welded. Such a structure is thus preferred.

As described above, in the hollow fiber membrane blood purifying device 1 of the embodiment, the hollow fiber membrane bundle 3 together with a peripheral portion thereof (an annular portion of the potting resin fixing portion 4 close to the inner peripheral wall of the cylindrical container 2) is concurrently, entirely cut into the same face, and the inside face of the header 5 is brought into pressure contact with the cut face (potting resin fixing portion cut face 4S). With such a structure, problems of conventional hollow fiber membrane blood purifying devices:
- a problem of blood retaining and activation arising from a groove formed between a level difference of a potting resin fixing portion and a header inside face; and
- a problem of invasion of blood between a header and a potting material can be avoided.

Moreover, in the hollow fiber membrane blood purifying device 1 of the embodiment, a shear joint 20 is adopted, and at least two areas including a contact area between the outer peripheral side face of the cylindrical container 2 and the inside face of the header 5 and a contact area between an end of the cylindrical container 2 and the inside face of the header 5 are welded, thereby achieving more sufficient fluid-tightness/air-tightness.

The above embodiments are preferred examples of the present invention, but the invention is not intended to be limited to the embodiments, and various modifications can be made without departing from the scope of the present invention.

### Examples 1

The present invention will next be described with reference to specific examples and comparative example, but is not intended to be limited to the following examples.

### <Hardness measurement of potting resin>

A hollow fiber membrane blood purifying device was disassembled into a cylindrical container and headers. Of a potting resin fixing portion, a cylindrical container peripheral part in which hollow fibers were not embedded and fixed was used as a measurement area, and a momentary value was determined with a D hardness tester.

### <Welded cross-sectional area>

An obtained hollow fiber membrane blood purifying device was subjected to X-ray-CT measurement at four positions over the circumferential direction. An area where a shear joint placed on a header or a cylindrical container interfered and the header and the cylindrical container were fused or deformed was identified, and the cross-sectional area thereof was calculated.

### <Height of potting resin fixing portion cut face>

After the ascertainment of curing a potting resin to embed and fix a hollow fiber membrane bundle 3 and a cylindrical container 2, an excess potting resin fixing portion 4 was cut and removed, and the heights of the potting resin fixing portion cut face was determined at four positions over the circumferential direction in accordance with JIS-B7517. The difference between a maximum value and a minimum value of the heights of the cut face (difference in height) was determined.

### <Contact width with header inside face>

A prepared hollow fiber membrane blood purifying device was subjected to X-ray-CT measurement to determine the widths at four positions over the circumferential direction (an interval of 90° in the circumferential direction).

### <Fluid-tightness evaluation: India ink test>

Through the fluid inlet and outlet nozzles of the headers and through the fluid inlet and outlet ports of the cylindrical container of a prepared hollow fiber membrane blood purifying device, a dialysate was charged, and to the fluid inlet and outlets of the cylindrical container, plugs were attached to make the device fluid-tight. Then, a 1% by weight India ink diluted with physiological saline was injected through the fluid inlet or outlet nozzle of a header to replace the flow path through the headers with the India ink, and circulation test was performed at Qb = 800 mL/min for 10 minutes. The maximum pressure at the inlet side was determined, and concurrently, soakage of the India ink between the header inside face and the potting resin fixing portion cut face was visually observed.

After the circulation test at Qb = 800 mL/min for 10 minutes, a compressed air was used to apply an internal pressure of 100 kPa. After 1 minute, soakage of the India ink between the header inside face and the potting resin fixing portion cut face was visually observed. The hollow fiber membrane blood purifying device after the completion of the test was further subjected to X-ray-CT measurement, and soakage of the India ink between the header inside face and the potting resin fixing portion cut face was observed.

### <Residual blood evaluation: bovine blood circulation test>

A prepared hollow fiber membrane blood purifying device was washed with 1.5 L of physiological saline, and then bovine fresh blood (a hematocrit value of 40%, a total protein content of 6.0 g/dl, an amount of heparin added of 2,500 IU/L) was circulated at a transmembrane pressure difference of 100 mmHg and a blood flow rate of 200 mL/min for 1 hour. For each hollow fiber membrane blood purifying device, 1.5 L of blood was used. Then, 400 ml of physiological saline was used to return the blood. The contact area between the potting resin fixing portion and the header inside face after the blood returning operation was visually observed to determine whether residual blood or blood aggregates were present.

### <Pressure-resistivity evaluation: pressure-resistance test>

The pressure at which a hollow fiber membrane blood purifying device was broken was determined in accordance with the following procedure. A drill was inserted through the fluid inlet and outlet nozzles of the headers of a prepared hollow fiber membrane blood purifying device to form a through-hole at a part of the potting resin fixing portion. Through the fluid inlet and outlet nozzles of the headers and the fluid inlet and outlet ports of the cylindrical container, water was charged, and plugs were attached to two fluid inlet and outlet nozzles of the headers and the fluid outlet of the cylindrical container. Then, through the fluid inlet of the cylindrical container, water was injected with a hydraulic pump to pressurize the device every 0.1 MPa to a maximum pressure of 2.0 MPa until the hollow fiber membrane blood purifying was broken.

### <Contact state between header and urethane>

A prepared hollow fiber membrane blood purifying device was disassembled into a cylindrical container and headers, and then a potting resin fixing portion cut face was observed. The rate of a hollow mark relative to the circumference of a potting resin fixing portion cut face was evaluated where the case in which a hollow mark by close contact with a header inside face was observed over the circumference of a potting resin fixing portion cut face was regarded as 100%, whereas the case in which any hollow mark by close contact with a header inside face was not observed was regarded as 0%.

### [Example 1]

A polypropylene resin cylindrical container having a shear joint-shaped interference portion over the circumference of an inside face of a concave shape including a flange portion on the outside face of the cylindrical container and polypropylene resin headers each having a shear joint-shaped interference portion over the circumference on the header inside face shown in Fig. 3 were used.

The inside face of each end of the cylindrical container was subjected to corona treatment under atmospheric pressure, and then a hollow fiber membrane bundle prepared by bundling 7,700 hollow fiber membranes having an inner diameter of 200 µm and a membrane thickness of 43 µm was inserted. A potting resin was injected before centrifugal rotation, curing, and storage, and the hollow fiber membrane bundle was embedded and fixed at each end of the cylindrical container, forming a potting resin fixing portion. A potting resin fixing portion protruding from each end of the cylindrical container was uniformly cut over the circumference to open each end of the hollow fiber membrane bundle. Here, the average height of the potting resin fixing portion cut face was 0.7 mm, and the difference in height on the potting resin fixing portion cut face was 0.2 mm.

The cylindrical container in which the potting resin fixing portion had been cut and the header were subjected to ultrasonic welding at a frequency of 20 kHz, a welding pressure of 0.3 MPa, a welding time of 0.15 second, and a holding time of 0.5 second.

The contact width between the header inside face and the potting resin fixing portion cut face was 0.8 mm at four positions in the circumferential direction of the obtained hollow fiber membrane blood purifying device.

The obtained hollow fiber membrane blood purifying device was used to perform India ink test. When circulation was performed at Qb = 800 mL/min for 10 minutes, the maximum pressure at the inlet side was 24 kPa. After 10 minutes of the circulation, no soakage of the India ink between the header inside face and the potting resin fixing portion cut face was observed. After the India ink test, an internal pressure of 100 kPa was applied. Even after 1 minute of the pressure application, no soakage of the India ink between the header inside face and the potting resin fixing portion cut face was observed.

X-ray-CT measurement was further performed, and no soakage of the India ink between the header inside face and the potting resin fixing portion cut face was observed.

The hollow fiber membrane blood purifying device after the India ink test was disassembled into the cylindrical container and the headers, and the potting resin fixing portion cut face was visually observed. On the potting resin fixing portion cut face, a contact mark of the header inside face was left over the circumference, and the contact state was 100%.

Of the potting resin fixing portion, a cylindrical container peripheral part in which hollow fibers were not embedded and fixed was subjected to hardness measurement with a D hardness tester to determine a momentary value, giving a hardness of 30.

A hollow fiber membrane blood purifying device prepared in the same manner as above was used to perform bovine blood circulation test. After 3 hours of the circulation test, residual blood or blood aggregates were not observed in the contact area between the header inside face and the potting resin fixing portion cut face.

A hollow fiber membrane blood purifying device prepared in the same manner as above was used to perform pressure-resistance test. The pressure resistance was 1.7 MPa.

### [Example 2 to Example 6]

The same procedure as in Example 1 was performed except that the header, the cylindrical container, and the potting resin were adjusted as described in Tables 1 and 2, giving hollow fiber membrane blood purifying devices. The evaluation results of the obtained hollow fiber membrane blood purifying devices are shown in Tables 1 and 2.

**[Table 1]**

| | | Example 1 | Example 2 | Example 3 | Example 4 |
|---|---|---|---|---|---|
| Header | Joint shape | Shear joint | Shear joint | Shear joint | Shear joint |
| | Header welding portion area /mm2 | 0.36 | 0.59 | 0.14 | 0.36 |
| Container | Presence or absence of flange portion | Presence | Presence | Presence | Presence |
| | Joint shape | Shear joint | Shear joint | Shear joint | Shear joint |
| | Container welding portion area /mm2 | 0.16 | 0.25 | 0.16 | 0.16 |
| Welding portion total area /mm2 | | 0.52 | 0.84 | 0.30 | 0.52 |
| Potting resin | Potting resin hardness | 30 | 30 | 30 | 70 |
| | Height of potting resin fixing portion cut face /mm | 0.7 | 0.3 | 1.1 | 0.7 |
| | Height difference of potting resin fixing portion /mm | 0.2 | 0.2 | 0.2 | 0.2 |
| Contact state between header and urethane /% | | 100 | 100 | 100 | 100 |
| Header inside face contact width /mm | | 0.8 | 0.8 | 0.8 | 0.8 |
| India ink test | Inlet side maximum pressure in circulation test /kPa | 24 | 25 | 21 | 23 |
| | Soakage of India ink, presence/absence | Absence | Absence | Absence | Absence |
| | Soakage of India ink after 100-kPa application, presence/absence | Absence | Absence | Absence | Absence |
| Bovine blood circulation test | Residual blood evaluation | No residual blood No blood aggregate | No residual blood No blood aggregate | No residual blood No blood aggregate | No residual blood No blood aggregate |
| Pressure-resistance test | Pressure resistance /MPa | 1.7 | - | 1.0 | 1.6 |

**[Table 2]**

| | | Example 5 | Example 6 | Example 7 | Example 8 | Example 9 |
|---|---|---|---|---|---|---|
| Header | Joint shape | Shear joint | Shear joint | Shear joint | Shear joint | Shear joint |
| | Header welding portion area /mm2 | 0.36 | 0.36 | 0.36 | 0.36 | 0.36 |
| Container | Presence or absence of flange portion | Presence | Presence | Absence | Presence | Presence |
| | Joint shape | Shear joint | Shear joint | Shear joint | Shear joint | Shear joint |
| | Container welding portion area /mm2 | 0.16 | 0.16 | 0.16 | 0.16 | 0.16 |
| Welding portion total area /mm2 | | 0.52 | 0.52 | 0.52 | 0.52 | 0.52 |
| Potting resin | Potting resin hardness | 30 | 30 | 30 | 30 | 30 |
| | Height of potting resin fixing portion cut face /mm | 0.7 | 0.7 | 0.7 | 0.7 | 0.7 |
| | Height difference of potting resin fixing portion /mm | 0.2 | 0.2 | 0.2 | 0.2 | 0.2 |
| Contact state between header and urethane /% | | 100 | 100 | 100 | 100 | 100 |
| Header inside face contact width/mm | | 1.5 | 0.4 | 0.8 | 0.8 | 0.8 |
| India ink test | Inlet side maximum pressure in circulation test /kPa | 23 | 22 | 22 | 2 | 22 |
| | Soakage of India ink, presence/absence | Absence | Absence | Absence | Absence | Absence |
| | Soakage of India ink after 100-kPa application, presence/absence | Absence | Absence | Absence | Absence | Absence |
| Bovine blood circulation test | Residual blood evaluation | No residual blood No blood aggregate | No residual blood No blood aggregate | No residual blood No blood aggregate | No residual blood No blood aggregate | No residual blood No blood aggregate |
| Pressure-resistance test | Pressure resistance /MPa | 1.7 | 1.7 | 1.8 | 1.9 | 1.1 |

### [Example 7]

The same procedure as in Example 1 was performed except that the flange portion on the cylindrical container outside face was removed by cutting, giving a hollow fiber membrane blood purifying device. The evaluation results of the obtained hollow fiber membrane blood purifying device are shown in Table 2.

### [Example 8]

The same procedure as in Example 1 was performed except that the shear joint-shaped interference portion on the inside face of the concave shape was moved to the side opposite to the inside face of the concave shape, giving a hollow fiber membrane blood purifying device. The evaluation results of the obtained hollow fiber membrane blood purifying device are shown in Table 2.

### [Example 9]

The same procedure as in Example 1 was performed except that the shear joint-shaped interference portion on the inside face of the concave shape was divided into 12 portions, and the portions were placed along the circumferential direction at equal intervals (discontinuous welding), giving a hollow fiber membrane blood purifying device. The evaluation results of the obtained hollow fiber membrane blood purifying device are shown in Table 2.

### [Comparative Example 1]

Unlike Examples 1 to 9, a polypropylene resin cylindrical container was used to form a potting resin fixing portion so as to form an annular potting resin fixing portion uncut face having a height of 0.7 mm and a width of 2.0 mm from the inside of the cylindrical container.

The potting resin fixing portion was cut at a height of 1.3 mm from each end of the cylindrical container to open each end of the hollow fiber membrane bundle, thereby forming a potting resin fixing portion uncut face having a height of 0.7 mm and a width of 2.0 mm from the inside of the cylindrical container and forming, on the inner peripheral side thereof, a potting resin fixing portion cut face having a height of 1.3 mm. Except the above, the same procedure as in Example 1 was performed to give a hollow fiber membrane blood purifying device. The evaluation results of the obtained hollow fiber membrane blood purifying device are shown in Table 3.

**[Table 3]**

| | | Comparative Example 1 | Comparative Example 2 | Comparative Example 3 |
|---|---|---|---|---|
| Header | Joint shape | Shear joint | Shear joint | Butt joint |
| | Header welding portion area /mm2 | 0.36 | 0.36 | 0.07 |
| Container | Presence or absence of flange portion | Presence | Presence | Presence |
| | Joint shape | Shear joint | Shear joint | Shear joint |
| | Container welding portion area /mm2 | 0.16 | 0.16 | 0.16 |
| Welding portion total area /mm2 | | 0.52 | 0.52 | 0.23 |
| Potting resin | Potting resin hardness | 30 | 30 | 30 |
| | Height of potting resin fixing portion cut face /mm | 1.3 | 1.1 | 0.7 |
| | Height difference of potting resin fixing portion /mm | 0.6 | 0.4 | 0.2 |
| Contact state between header and urethane /% | | 100 | 100 | 80 |
| Header inside face contact width /mm | | 0.8 | 0.8 | 0.8 |
| India ink test | Inlet side maximum pressure in circulation test/kPa | 21 | 20 | 21 |
| | Soakage of India ink, presence/absence | Absence | Absence | Presence |
| | Soakage of India ink after 100-kPa application, presence/absence | Absence | Absence | Presence |
| Bovine blood circulation test | Residual blood evaluation | Ring-like residual blood Blood aggregates | Ring-like residual blood | No residual blood No blood aggregate |
| Pressure-resistance test | Pressure resistance /MPa | 1.7 | 1.7 | 0.7 |

In the India ink test of the obtained hollow fiber membrane blood purifying device, no soakage of the India ink between the header inside face and the potting resin fixing portion cut face 4S' was observed, but in the bovine blood circulation test, ring-like residual blood and blood aggregates were observed at the time of blood returning in the groove portion formed between the header inside face and the potting resin fixing portion cut face (see Fig. 4).

### [Comparative Example 2]

In the same manner as in Comparative Example 1, a polypropylene resin cylindrical container was used to form a potting resin fixing portion, and the potting resin fixing portion uncut face was adjusted to have a height of 0.7 mm. After curing, the potting resin fixing portion was cut at a position 1.1 mm apart from each end of the cylindrical container to open each end of the hollow fiber membrane bundle, thereby forming a potting resin fixing portion uncut face having a height of 0.7 mm on the outer peripheral part of the cylindrical container and forming, on the inner periphery thereof, a potting resin fixing portion cut face having a height of 1.1 mm. Except the above, the same procedure as in Example 1 was performed to give a hollow fiber membrane blood purifying device. The evaluation results of the obtained hollow fiber membrane blood purifying device are shown in Table 3.

In the India ink test of the obtained hollow fiber membrane blood purifying device, no soakage of the India ink between the header inside face and the potting resin fixing portion cut face was observed, but in the bovine blood circulation test, ring-like residual blood was observed at the time of blood returning.

### [Comparative Example 3]

The same procedure as in Example 1 was performed except that the joint shape of the header was changed to a butt joint having an energy director, giving a hollow fiber membrane blood purifying device. The evaluation results of the obtained hollow fiber membrane blood purifying device are shown in Table 3.

In the India ink test of the obtained hollow fiber membrane blood purifying device, soakage of the India ink between the header inside face and the potting resin fixing portion cut face was observed. The hollow fiber membrane blood purifying device was disassembled, and the contact state between the header and urethane was observed to be 80%, which indicated that the urethane was not compressively deformed in some areas in the circumferential direction.

### Industrial Applicability

The present invention is suitable for a hollow fiber membrane blood purifying device that uses hollow fiber membranes packed in a main body container to purify blood.

### Reference Signs List

1 hollow fiber membrane blood purifying device
2 cylindrical container
2a one end of a cylindrical container
2b the other end of the cylindrical container
2c side face
3 hollow fiber membrane
3a, 3b each end
4 potting resin fixing portion
4S potting resin fixing portion cut face
5 header
6 (6a, 6b) nozzle
7 (7a, 7b) port
20 shear joint
20h concave portion
20t convex portion
21, 22 interference portion
31, 32welding portion
45 contact face
51 inner peripheral edge of a header
52 contact face of a header

## Claims

1. A hollow fiber membrane blood purifying device comprising:
a cylindrical container having one open end and another open end;
a hollow fiber membrane bundle packed in the cylindrical container;
a potting resin fixing portion embedding and fixing the hollow fiber membrane bundle at each end of the cylindrical container;
a header provided at each end of the cylindrical container and having a nozzle serving as an outlet or inlet of a fluid; and
a port provided on a side of the cylindrical container and serving as an outlet or inlet of a fluid passing through the hollow fiber membrane, wherein
a shear joint is used as a joint design for welding,
the device has a structure in which the header and the cylindrical container are welded in at least two or more regions,
an inside face of the header is in contact with a potting resin fixing portion cut face that is formed by cutting an outer side portion of the potting resin fixing portion in a length direction of the cylindrical container than an end face of each end of the cylindrical container, and the potting resin fixing portion cut face is in a compressively deformed state.

2. The hollow fiber membrane blood purifying device according to claim 1, wherein the welded area includes a continuous or discontinuous area over a circumference on a side face on an outer periphery of the cylindrical container and on the inside face of the header and a continuous or discontinuous area over a circumference on each end or a side face of the end of the cylindrical container and on the inside face of the header.

3. The hollow fiber membrane blood purifying device according to claim 1 or 2, wherein a contact face on the inside face of the header in contact with the potting resin fixing portion cut face has a flat face extending outwardly from an inner peripheral edge along a face substantially parallel with the potting resin fixing portion cut face.

4. The hollow fiber membrane blood purifying device according to any one of claims 1 to 3, wherein, in a longitudinal section where the header and the cylindrical container are welded, a total sum of a cross-sectional area where the header inside face and the cylindrical container are welded is 0.05 to 1.25 mm².

5. The hollow fiber membrane blood purifying device according to any one of claims 1 to 4, wherein, in a longitudinal section where the header and the cylindrical container are welded, a contact width between the potting resin fixing portion cut face and the header inside face is 0.3 to 1.5 mm.

6. The hollow fiber membrane blood purifying device according to any one of claims 1 to 5, wherein, in a longitudinal section where the header and the cylindrical container are welded, a height from the end face of the cylindrical container to the cut face of the potting resin fixing portion ranges from 0.1 to 1.5 mm.

7. The hollow fiber membrane blood purifying device according to any one of claims 1 to 6, wherein a raw material of the cylindrical container and the headers is a polypropylene resin.

8. The hollow fiber membrane blood purifying device according to any one of claims 1 to 7, wherein the potting resin has a hardness determined by a Shore type D durometer of 30 to 70.

9. The hollow fiber membrane blood purifying device according to any one of claims 1 to 8, wherein, in a longitudinal section of the header and the cylindrical container, one of the header and the cylindrical container has a convex portion with a convex shape, the other has a concave portion with a concave shape to which the convex portion with the convex shape is fit, the convex portion has a leading end face width larger than a bottom face width of the concave portion, and at least one side face of inside faces of the concave portion has a shear joint design with a slope that makes the concave portion bottom face width smaller than a concave portion top face width.
